(19) Europäisches Patentamt
European Patent Office
Office européen des brevets

(11) EP 1 268 499 B1

(12) EUROPÄISCHE PATENTSCHRIFT

(45) Veröffentlichungstag und Bekanntmachung des Hinweises auf die Patenterteilung:
**12.10.2005 Patentblatt 2005/41**

(21) Anmeldenummer: **01915377.4**

(22) Anmeldetag: **19.03.2001**

(51) Int Cl.[7]: **C07F 15/00**, C07C 6/00, C08F 253/00, C08F 255/06, C08F 257/02

(86) Internationale Anmeldenummer:
**PCT/EP2001/003118**

(87) Internationale Veröffentlichungsnummer:
**WO 2001/072760 (04.10.2001 Gazette 2001/40)**

# (54) VERFAHREN ZUR POLYMERISATION VON POLAR SUBSTITUIERTEN CYCLOALKENEN

METHOD FOR POLYMERIZING POLAR SUBSTITUTED CYCLOALKENES

PROCEDE DE POLYMERISATION DE CYCLOALKENES SUBSTITUES DE MANIERE POLAIRE

(84) Benannte Vertragsstaaten:
**DE FR GB IT NL**

(30) Priorität: **29.03.2000 DE 10015452**

(43) Veröffentlichungstag der Anmeldung:
**02.01.2003 Patentblatt 2003/01**

(73) Patentinhaber: **LANXESS Deutschland GmbH**
**51369 Leverkusen (DE)**

(72) Erfinder:
- **OSTOJA STARZEWSKI, Karl-Heinz, Aleksander**
  **61118 Bad Vilbel (DE)**
- **WEISS, Karin**
  **95463 Bindlach (DE)**
- **THÜRING, Martin, Olaf**
  **95615 Marktredwitz (DE)**

(56) Entgegenhaltungen:
**US-A- 4 039 491**

- **WEISS K ET AL: "UNTERSUCHUNGEN VON POLYMERISATIONS- UND METATHESE-REAKTIONEN" JOURNAL OF ORGANOMETALLIC CHEMISTRY,CH,ELSEVIER-SEQUOIA S.A. LAUSANNE, Bd. 355, Nr. 1 - 03 + INDEX, 8. November 1988 (1988-11-08), Seiten 273-280, XP000006804 ISSN: 0022-328X**
- **RICHARD R. SCHROCK: "Metathesis reactions catalyzed by well-characterized transition metal alkylidene complexes" THE TREM CHEMIKER, Bd. XIV, Nr. 1, 1992, Seiten 1-6, XP001005690**
- **K.WEISS, R.GOLLER, M.DENZNER, L.LÖSSEL, J.KÖDEL: "Transition Metal Carbyne Complexes" 1993 , F.R.KREISSEL, KLUWER , DODRECHT, NL XP002170686 Seite 55 -Seite 66; Tabellen 2,3**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 268 499 B1

Printed by Jouve, 75001 PARIS (FR)

## Beschreibung

Die vorliegende Erfindung betrifft ein Verfahren zur (Co-)Polymerisation von polar substituierten Norbornenen und/oder Cycloalkenen gegebenfalls in Anwesenheit einer weiteren ungesättigten Verbindung, dadurch gekennzeichnet, dass in Gegenwart einer oder mehrerer Wolfram-Carbin-Komplexe und/oder Molybdän-Carbin Komplexe polymerisiert wird.

Cycloolefin-(Co)Polymere zeichnen sich durch viele vorteilhafte Eigenschaften aus, so durch eine hohe Transparenz zum Einsatz für optische Anwendungen. Sie haben eine gute thermische Beständigkeit, Alterungsbeständigkeit, chemische Beständigkeit, gute Gasbarriereeigenschaften, Lösungsmittelbeständigkeit, geringe Wasseraufnahme, hohe Kratzfestigkeit, geringe Lichtdoppelbrechung und hohe Erweichungstemperaturen (Glasübergangstemperaturen $T_g$). Solche (Co)Polymere sind daher u.a. geeignet für: Folien in unverstreckter oder monoaxial oder biaxial verstreckter Form für Verpackungen und als Deckschichten für Polarisationsfolien und Flüssigkristalldisplays als Materialien für optische Datenspeicher, Lackbestandteile, etwa für die Automobilindustrie zur Kratzfestausrüstung von Oberflächen; Fasern, etwa für Lichtwellenleiter; optische Linsen und Prismen; Schläuche, Rohre, Stangen und Trägerplatten; Deckscheiben für Solarzellen; Kondensator-Dielektrikum. Solche technischen Artikel werden durch Spritzguss oder Extrusion hergestellt. Die so eingesetzten (Co)Polymere sind amorph oder nur teilkristallin. Die (Co)Polymere können allein oder in Abmischung mit anderen Polymeren angewandt werden.

Die Polymerisation von cyanosubstituierten Norbornenderivaten ist aus DE-A-2 316 087 prinzipiell bekannt. Als Katalysatoren für die ringöffnende Polymerisation der Norbornene werden Wolfram- und Molybdänverbindungen, insbesondere deren Halogenide, in Verbindung mit Aluminiumalkylen eingesetzt. Es werden keine Metall-Carbin-Komplexe offenbart. Die hier offenbarten Ziegler-Natta-Katalysatoren sind in Gegenwart polarer Gruppen nicht ausreichend aktiv.

US-A-4,039,491 offenbart Norbornen-Polymere und Copolymere hergestellt aus Norbornenmonomeren unter Verwendung von Wolfram- und Molybdänverbindungen und metallorganischen Verbindungen der Gruppen 1, 2, 12 und 13 des Periodensystems nach IUPAC 1985. Es werden keine Metall-Carbin-Komplexe offenbart. Die hier offenbarten Ziegler-Natta-Katalysatoren sind in Gegenwart polarer Gruppen nicht ausreichend aktiv.

US-A-5,312,940 offenbart Cycloolefinpolymerisate und deren Herstellung mit Metall-Carben-Komplexen als Katalysatoren. Metall-Carbin-Komplexe werden nicht genannt. Zudem sind die genannten Carben-Komplexe aufwendig zu synthetisieren.

DE-A1-198 15 275 offenbart ebenfalls Cycloolefinpolymerisate und deren Herstellung mit Metall-Carben-Komplexen als Katalysatoren. Metall-Carbin-Komplexe werden auch hier nicht genannt. Die genannten Carben-Komplexe sind ebenfalls nur aufwendig zu synthetisieren.

K. Weiss, R. Goller, M. Denzner, G. Lößel, J. Ködel in *Transition Metal Carbyne Complexes,* (Hrsg.: F.R. Kreißl), Kluwer, Dordrecht, **1993**, 55 offenbaren die Polymerisation von nicht-polar substituierten Cycloalkenen mit Wolfram-Carbinkomplexen. Die Polymerisation von polar substituierten Cycloalkenen wird nicht beschrieben.

Es bestand also die Aufgabe, hochwirksame Metathesekatalysatoren zur Homo- und Copolymerisation von polar substituierten Cycloolefinen insbesondere polar substituierten Norbornenen zu finden.

Weiterhin bestand die Aufgabe die Nachteile der im Stand der Technik bekannten Verfahren wenigstens teilweise zu vermeiden.

Weiterhin bestand die Aufgabe, alternative Methathesekatalysatoren zur Homo- und Copolymerisation von polar substituierten Cycloolefinen insbesondere polar substituierten Norbornenen zu finden.

Die Aufgaben werden erfindungsgemäss gelöst durch die Bereitstellung eines Verfahrens zur Polymerisation von polar substituierten Cycloalkenen gegebenfalls in Anwesenheit einer oder mehreren weiteren ungesättigten Verbindungen, dadurch gekennzeichnet, dass in Gegenwart einer oder mehrerer Wolfram-Carbin-Komplexe und/oder Molybdän-Carbin-Komplexe polymerisiert wird.

Cycloalkene sind mono- oder polycyclisch und fallen unter eine der beiden Formeln

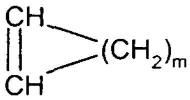 ( I ) bzw. 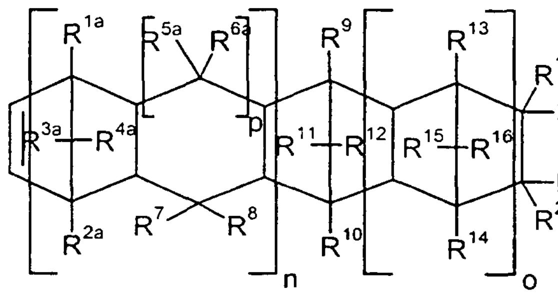 ( II ),

in denen die Indices

m eine Zahl von 2 bis 10, bevorzugt 3 bis 6,

n die Zahl 0 oder 1,

o die Zahl 0, 1, 2 oder 3 und

p die Zahl 0 oder 1 bedeutet,

in Formel (I) zwei benachbarte $CH_2$-Gruppen durch die Gruppe -CH=CH- ersetzt sein kann und in Formel (II) die Reste $R^{1a}$ bis $R^{6a}$ und $R^7$ bis $R^{20}$ unabhängig voneinander Wasserstoff, Fluor, Chlor, Brom, geradkettiges oder verzweigtes $C_1$-$C_{20}$-Alkyl, $C_3$-$C_8$-Cycloalkyl oder $C_6$-$C_{16}$-Aryl darstellen, wobei zusätzlich das Restepaar $R^{18}/R^{19}$ eine Doppelbindung oder eine der Gruppen $-CHR^{21}-CHR^{22}-CHR^{23}-$, $-CHR^{21}-CHR^{22}-CHR^{23}-CHR^{24}-$ oder $-CHR^{21}-CHR^{22}-CHR^{23}-CHR^{24}-CHR^{25}-$, in denen $R^{21}$ bis $R^{25}$ Wasserstoff oder $C_1$-$C_4$-Alkyl sind, bedeuten kann und das Restepaar $R^{17}/R^{18}$ die doppelt gebundene Gruppe $=C(R^{26},R^{27})$, in der $R^{26}$ und $R^{27}$ $C_1$-$C_4$-Alkyl sind und $R^{27}$ auch Wasserstoff sein kann, bedeuten kann, wobei ein oder mehrere Reste durch einen polaren Rest ausgewählt aus der Gruppe -CN, Halogen, $-CF_3$, $-C_2F_5$ bis $C_{20}$-$F_{41}$, -N($C_1$-$C_{30}$-Alkyl)$_2$, $C_1$-$C_{12}$-Alkoxy oder $C_1$-$C_{20}$-Alkylen-$COOC_1$-$C_{20}$-alkyl, -OH, -OR ersetzt sind. Selbstverständlich können die Verbindungen auch mehrere gegebenenfalls unterschiedliche polare Substituenten enthalten.

Solche Cycloalkene haben eine oder mehrere, bevorzugt eine oder zwei Doppelbindungen und sind bekannt und beispielsweise in den Verfahren von EP-A 610 852, EP-A 690 078 und US 5 567 777 eingesetzt.

Bevorzugte Cycloalkene der Formel (II) sind solche der Formeln

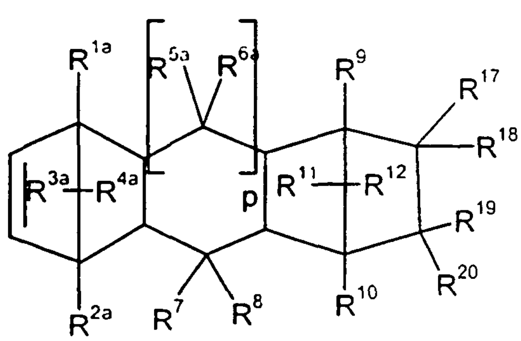

( II a )

bzw.

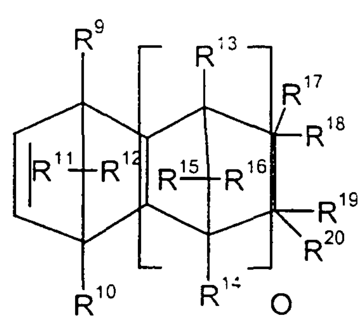

( II b ).

wobei die Reste R und die Indizes die bereits genannten Bedeutungen haben, wobei bei diesen genannten Grundkörper an beliebigen Stellen ein oder mehrere Reste durch eine polare Gruppe ausgewählt aus der Gruppe -CN, Halogen, $-CF_3$, $-C_2F_5$ bis $C_{20}$-$F_{41}$, -N($C_1$-$C_{30}$-Alkyl)$_2$, $C_1$-$C_{12}$-Alkoxy oder $C_1$-$C_{20}$-Alkylen-$COOC_1$-$C_{20}$-alkyl, -OH, -OR ersetzt ist. Selbstverständlich können die Verbindungen auch mehrere polare gegebenenfalls unterschiedliche Substituenten enthalten.

Eine beispielhafte, nicht erschöpfende Aufzählung solcher Grund-Cycloalkene umfasst Cyclobuten, Cyclopenten, Cyclopentadien, Cyclohexen, Cyclohepten, Cyclooctten, Cyclodecen, Cyclododecen, Bicyclo-2-heptene, Tricyclo-3-decene, Tricyclo-3-undecene, Tetracyclo-3-dodecene, Pentacyclo-4-pentadecene, Pentacyclopentadecadiene, Pentacyclo-3-pentadecene, Pentacyclo-4-hexadecene, Pentacyclo-3-hexadecene, Hexacyclo-4-heptadecene, Heptacyclo-5-eicosene, Heptacyclo-4-eicosene, Heptacyclo-5-heneicosene, Octacyclo-5-docosene, Nonacyclo-5-pentacosene, Nonacyclo-6-hexacosene, Cyclopentadien/Acenaphthylen-Addukte, 1,4-Methano-1.4.4a.9a-tetrahydrofluorene und 1,4-Methano-1.4.4a.5.10.10a-hexahydroanthracene, wie Bicyclo[2,2,1]-hept-2-en (Norbornen), Norbornadien, 5-Methyl-norbornen, 6-Methyl-norbornen, 5,6-Dimethyl-norbornen, 1-Methyl-norbornen, 5-Isobutyl-norbornen, 7-Methyl-norbornen, Tricyclo[4,3,0,$1^{2,5}$]-3-decen (5,6-Trimethylen-norbornen), Tricyclo-[4,4,0,$1^{2,5}$]-3-undecen (5,6-Tetramethylen-norbornen), 10-Methyl-tricyclo-[4,4,0,$1^{2,5}$]-3-undecen, 6-Ethylbicyclo[2.2.1]hept-2-en, 6-n-Butylbicyclo[2.2.1]hept-2-en, 6-Isobutylbicyclo[2.2.1]hept-2-en, 2-Methyltricyclo[4.3.0.$1^{2,5}$]-3-decen, 5-Methyltricyclo [4.3.0.$1^{2,5}$]-3-decen, Tricyclo[4.3.0.$1^{2,5}$]-3-undecen, Tricyclo[4,3,0,$1^{2,5}$]-3,7-decadien (Dicyclopentadien), Trlcyclo [4,3,0,$1^{2,5}$]-3-decen, Tetracyclo-[4,4,0,$1^{2,5}$,$1^{7,10}$]-3-dodecen, 8-Methyl-tetracyclo[4,4,0,$1^{2,5}$,$1^{7,10}$]-3-dodecen, 8-Cyclohexyl-tetracyclo[4,4,0,$1^{2,5}$,$1^{7,10}$]-3-dodecen, 8-Stearyl-tetracyclo-[4,4,0,$1^{2,5}$,$1^{7,10}$]-3-dodecen, das 5,10-Dimethyl-, 2,10-Dimethyl-, 8,9-Dimethyl-, 11,12-Dimethyl-, 2,7,9-Trimethyl-, 9-Isobutyl-, 11,12-Dimethyl-, 8-Ethyliden-9-methyl, 8-Chlor-, 8-Brom- oder 8-Fluor-derivat des Tetracyclo[4,4,0,$1^{2,5}$,$1^{7,10}$]-3-dodecen, 8-Ethyltetracyclo[4.4.0.$1^{2,5}$.$1^{7,10}$]-3-dodecen, 8-Propyltetracyclo[4.4.0.$1^{2,5}$.$1^{7,10}$]-3-dodecen, 8-Butyltetracyclo[4.4.0.$1^{2,5}$.$1^{7,10}$]-3-dodecen, 8-Isobutyltetracyclo-[4.4.0.$1^{2,5}$.$1^{7,10}$]-3-dodecen, 8-Hexyltetracyclo[4.4.0.$1^{2,5}$.$1^{7,10}$]-3-dodecen, 8-Methyl-9-ethyltetracyclo [4.4.0.$1^{2,5}$.$1^{7,10}$]-3-dodecen. 9-Ethyl-2,7-dimethyltetracyclo[4.4.0.$1^{2,5}$.$1^{7,10}$]-3-dodecen, 9-Isobutyl-2,7-dimethyltetracyclo[4.4.0.$1^{2,5}$.$1^{7,10}$]-3-dodecen, 9,11,12-Trimethyltetracyclo[4.4.0.$1^{2,5}$.$1^{7,10}$]-3-dodecen, 9-Ethyl-11,12-dimethyltetracyclo-[4.4.0.$1^{2,5}$.$1^{7,10}$]-3-dodecen, 9-Isobutyl-11,12-dimethyltetracyclo[4.4.0.$1^{2,5}$.$1^{7,10}$]-3-dodecen, 5,8,9,10-Tetramethyltetracyclo[4.4.0.$1^{2,5}$.$1^{7,10}$]-3-dodecen, 8-Ethylidentetracyclo[4.4.0.$1^{2,5}$.$1^{7,10}$]-3-dodecen, 8-Ethyliden-9-methyltetracyclo[4.4.0.$1^{2,5}$.$1^{7,10}$]-3-dodecen, 8-Ethyliden-9-ethyltetracyclo[4.4.0.$1^{2,5}$.$1^{7,10}$]-3-dodecen, 8-Ethyliden-9-isopropyltetracyclo[4.4.0.$1^{2,5}$.$1^{7,10}$]-3-dodecen, 8-Ethyliden-8-butyltetracyclo[4.4.0.$1^{2,5}$.$1^{7,10}$]-3-dodecen, 8-n-Propylidentetracyclo[4.4.0.$1^{2,5}$.$1^{7,10}$]-3-dodecen, 8-n-Propyliden-9-methyltetracyclo[4.4.0.$1^{2,5}$.$1^{7,10}$]-3-dodecen, 8-n-Propyliden-9-ethyltetracyclo[4.4.0.$1^{2,5}$.$1^{7,10}$]-3-dodecen, 8-n-Propyliden-9-isopropyltetracyclo[4.4.0.$1^{2,5}$.$1^{7,10}$]-3-dodecen, 8-n-Propyliden-9-butyltetracyclo[4.4.0.$1^{2,5}$.$1^{7,10}$]-3-dodecen, 8-Isopropylidentetracyclo[4.4.0.$1^{2,5}$.$1^{7,10}$]-3-dodecen, 8-Isopropyliden-9-methyltetracyclo-[4.4.0.$1^{2,5}$.$1^{7,10}$]-3-dodecen, 8-Isopropyliden-9-ethyltetracyclo[4.4.0.$1^{2,5}$.$1^{7,10}$]-3-dodecen, 8-Isopropyliden-9-isopropyltetracyclo[4.4.0.$1^{2,5}$.$1^{7,10}$]-3-dodecen, 8-Isopropyliden-9-butyltetraeyclo[4.4.0.$1^{2,5}$.$1^{7,10}$]-3-dodecen, 8,9-Dichlortetracyclo[4.4.0.$1^{2,5}$.$1^{7,10}$]-3-dodecen, Pentacyclo[6,5,1,$1^{3,6}$,$0^{2,7}$,$0^{9,13}$]-4-pentadecen, Pentacyclo-[7,4,0,$1^{2,5}$,$1^{9,12}$,$0^{8,13}$]-3-pentadecen, Pentacyclo[8,4,0,$1^{2,5}$,$1^{9,12}$,$0^{8,13}$]3-hexadecen, 1,3-Dimethylpentacyclo[6.5.1.$1^{3,6}$.$0^{2,7}$.$0^{9,13}$]-4-pentadecen, 1,6-Dimethyl[6.5.1.$1^{3,6}$.$0^{2,7}$.$0^{9,13}$]-4-pentadecen, 14,15-Dimethyl[6.5.1.$1^{3,6}$.$0^{2,7}$.$0^{9,13}$]-4-pentadecen, Pentacyclo-[7.4.0.$1^{2,5}$.$1^{9,12}$.$0^{8,13}$]-3-pentadecen, Methyl-substituierte Pentacyclo[7.4.0.$1^{2,5}$.$1^{9,12}$.$0^{8,13}$]-3-pentadecene, Pentacyclo[6.5.1.$1^{3,6}$.$0^{2,7}$.$0^{9,13}$]-4,10-pentadecadien, 11-Methylpentacyclo[8.4.0.$1^{2,5}$.$1^{9,12}$.$0^{8,13}$]-3-hexadecen, 11-Ethyl[8.4.0.$1^{2,5}$.$1^{9,12}$.$0^{8,13}$]-3-hexadecen, 10,11-Dimethyl[8.4.0.$1^{2,5}$.$1^{9,12}$.$0^{8,13}$]-3-hexadecen, Pentacyclo[6.6.1.$1^{3,6}$.$0^{2,7}$.$0^{9,14}$]-4-hexadecen, 1,3-Dimethylpentacyclo[6.6.1.$1^{3,6}$.$0^{2,7}$.$0^{9,14}$]-4-hexadecen, 15,16-Dimethylpentacyclo[6.6.1.$1^{3,6}$.$0^{2,7}$.$0^{9,14}$]-4-hexadecen, Hexacyclo-[6,6,1,$1^{3,6}$,$1^{10,13}$,$0^{2,7}$,$0^{9,14}$]-heptadecen, Heptacyclo-[8,7,0,$1^{2,9}$,$1^{4,7}$,$1^{11,17}$,$0^{3,8}$,$0^{12,16}$]-5-eicosen, Heptacyclo[8,8,0,$1^{4,7}$,$1^{11,18}$,$1^{13,16}$,$0^{3,8}$, $0^{12,17}$]-5-heneicosen, 12-Methylhexacyclo[6.6.1.$1^{3,6}$.$1^{10,13}$.$0^{2,7}$.$0^{9,14}$]-4-heptadecen, 12-Ethylhexacyclo[6.6.1.$1^{3,6}$.$1^{10,13}$.$0^{2,7}$.$0^{9,14}$]-4-heptadecen, 12-Isobutylhexacyclo[6.6.1.$1^{3,6}$.$1^{10,13}$.$0^{2,7}$.$0^{9,14}$]-4-heptadecen, 1,6,10-Trimethylhexacyclo[6.6.1.$1^{3,6}$.$1^{10,13}$.$0^{2,7}$.$0^{9,14}$]-4-heptadecen, Heptacyclo[8.7.0.$1^{3,6}$.$1^{10,17}$.$1^{12,15}$.$0^{2,7}$.$0^{11,16}$]-4-eicosen und seine dimethylsubstituierten Derivate, Heptacyclo[8.8.0.$1^{4,7}$.$1^{11,18}$.$1^{13,16}$.$0^{3,8}$.$0^{12,17}$]-5-heneicosen und seine trimethylsubstituierten Derivate, 15-Methylheptacyclo[8.8.0.$1^{4,7}$.$1^{11,18}$.$1^{13,16}$.$0^{3,8}$.$0^{12,17}$]-5-heneicosen, 5-Phenyl-bicyclo[2.2.1]hept-2-en, 5-Methyl-5-phenyl-bicyclo[2.2.1]hept-2-en, 5-Benzyl-bicyclo[2.2.1]hept-2-en, 5-Tolyl-bicyclo-[2.2.1]hept-2-en, 2-(Ethylphenyl)-bicyclo[2.2.1]hept-2-en, 5-(Isopropylphenyl)-bicyclo[2.2.1]hept-2-en, 5-Biphenyl-bicyclo[2.2.1]hept-2-en, 5-(β-Naphthyl)-bicyclo[2.2.1]hept-2-en, 5-(α-Naphthyl)-bicyclo[2.2.1]hept-2-en, 5-(Anthracenyl)-bicyclo[2.2.1]hept-2-en, 5,6-Diphenyl-bicyclo[2.2.1]hept-2-en, 1,4-Methano-1.4.4a.9a-tetrahydrofluoren, 1,4-Methano-1.4.4a.5.10.10a-hexahydroanthracen, 8-Phenyltetracyclo[4.4.0.$1^{2,5}$.$1^{7,10}$]-3-dodecen, 8-Methyl-8-phenyltetracyclo[4.4.0.$1^{2,5}$.$1^{7,10}$]-3-dodecen, 8-Benzyl-tetracyclo[4.4.0.$1^{2,5}$.$1^{7,10}$]-3-dodecen, 8-Tolyl-tetracyclo[4.4.0.$1^{2.5}$.$1^{7,10}$]-3-dodecen, 8-(Ethylphenyl)-tetracyclo[4.4.0.$1^{2,5}$.$1^{7,10}$]-3-dodecen, 8-(Isopropylphenyl)-tetracyclo[4.4.0.$1^{2,5}$.$1^{7,10}$]-3-dodecen, 8,9-Diphenyl-tetracyclo[4.4.0.$1^{2,5}$.$1^{7,10}$]-3-dodecen, 8-(Biphenyl)-tetracyclo[4.4.0.$1^{2,5}$.$1^{7,10}$]-3-dodecen, 8-(β-Naphthyl)-tetracyclo [4.4.0.$1^{2,5}$.$1^{7,10}$]-3-dodecen, 8-(α-Naphthyl)-tetracyclo[4.4.0.$1^{2,5}$.$1^{7,10}$]-3-dodecen und 8-(Anthracenyl)-tetracyclo [4.4.0.$1^{2,5}$.$1^{7,10}$]-3-dodecen, wobei wobei bei diesen genannten Grundkörper an beliebigen Stellen ein oder mehrere Reste durch eine polare Gruppe ausgewählt aus der Gruppe -CN ersetzt ist. Selbstverständlich können die Verbindungen auch mehrere polare Substituenten enthalten.

Bevorzugte Cycloalkene sind auch solche, die durch Halogen, $-CF_3$, $-C_2F_5$-$C_{20}F_{41}$, -N($C_1$-$C_{30}$-Alkyl)$_2$ -CN oder $C_1$-$C_{20}$-Alkylen-$COOC_1C_{20}$-alkyl, Hydroxy-, Hydroxyalkyl, vorzugsweise ein- bis dreimal pro Molekül substituiert sind. Besonders bevorzugte polar substituierte Cycloalkene sind Nitrilnorborne, wie 5-Cyano-2-norbornen und 5-Cyano-5-octyl-2-norbornen.

Die Cycloalkene können gegebenenfalls in Gegenwart von ungesättigten Verbindungen, insbesondere nicht polar substituierten Cycloalkenen, acyclischen Mono- oder Diolefinen, Alkinen polymerisiert werden.

Geeignete nicht polar substituierte Cycloalkene sind sämtliche genannte polar substituierte Cycloalkene, mit der Maßgabe, dass keine Reste R durch polare Gruppen ersetzt worden sind. Geeignete acyclische Olefine umfassen $C_2$-$C_{40}$-α-Olefine und nicht-konjugierte Diolefine, wie z.B. Ethylen, Propylen, 1-Buten, 1-Penten, 1-Hexen, 3-Methyl-1-buten, 3-Methyl-1-penten, 4-Methyl-1-penten, 4-Methyl-1-hexen, 4,4-Dimethyl-1-hexen, 4,4-Dimethyl-1-penten, 4-Ethyl-1-hexen, 3-Ethyl-1-hexen, 1-Octen, 1-Decen, 1-Dodecen, 1-Tetradecen, 1-Hexadecen, 1-Octadecen, 1-Eicosen und Mischungen dieser α-Olefine sowie 1,5-Hexadien, 1,6-Heptadien, 1,6- und 1,7-Octadien, 1,8-Nonadien, 1,9-Decadien, 1,11-Dodecadien, 1,19-Eicodien und Mischungen dieser Diolefine. Auch Mischungen von α-Olefinen und Diolefinen sind geeignet.

Solche Olefine und Diolefine können weiterhin substituiert sein, beispielsweise mit Phenyl, substituiertem Phenyl, Halogen, der veresterten Carboxylgruppe, der Säureanhydridgruppe; Verbindungen dieser Art sind beispielsweise Chloropren, Styol, Methylstyrol, Chlorstyrol, Fluorstyrol, Inden, 4-Vinyl-biphenyl, Vinyl-fluoren, Vinylanthracen, Methylmethacrylat, Ethylacrylat, Vinylsilan, Trimethylallylsilan, Vinylchlorid, Vinylidenchlorid, Tetrafluorethylen, Isobutylen, Vinylcarbazol, Vinvlpyrrolidon, Acrylnitril, Vinylether und Vinylester. Bevorzugte Monomere sind: Ethylen, Propylen, Buten, Hexen, Octen, 1,5-Hexadien, 1,6-Octadien, Methylmethacrylat und Acetylen.

Bevorzugt sind Ethylen und Propylen.

Das polar substituierte Cycloalken der Formeln (I), bzw. (II) stellt einen molaren Anteil von 1 bis 100 % der Gesamtmolzahl aller eingesetzten Comonomere. Das ungesättigte Monomer stellt einen molaren Anteil von 99 bis 0 % der Gesamtmolzahl aller eingesetzter Comonomerer. Die bevorzugten Mengen von polar substituiertem Cycloalken zu ungesättigtem Monomer betragen 20:80 mol-% bis 80:20 mol-%. Für den Fall, dass polar substituierte Cycloalkene sowohl der Formel (I) als auch der Formel (II) eingesetzt werden, beträgt deren molares Verhältnis 10:90 mol-% bis 90:10 mol-%. Die Co-polymerisation von polar und nicht polar substituierten Cycloalkenen erlaubt überraschenderweise die fast vollständige Umsetzung der Monomeren auch bei relativ hohen Monomer-Katalysator-Verhältnissen.

Das erfindungsgemäße Verfahren wird homogen bei Temperaturen im Bereich von -80° bis +200° und heterogen bei Temperaturen von -20° bis +150°C und Drücken im Bereich von 0,5 bis 500 bar in der Gas-, Masse-, Flüssig- oder in der Slurry-Phase durchgeführt, je nachdem, ob ein löslicher oder ein unlöslicher Katalysator der oben beschriebenen Art eingesetzt wird. Die Flüssigphase bzw. die Slurry-Phase kann aus den Comonomeren allein, d.h. ohne Verwendung eines zusätzlichen Lösungsmittels/Suspensionsmittels, gebildet werden. Für den Fall, dass ein Lösungsmittel mitverwendet wird, kommen hierfür inerte Lösungsmittel, beispielsweise aliphatische oder cycloaliphatische Kohlenwasserstoffe, Benzin- bzw. Dieselölfraktionen (gegebenenfalls nach einer Hydrierung), Toluol, Chlorbenzol, o-Dichlorbenzol, 1,2,4-Trichlorbenzol, THF, Diethylether, Chloroform, Methylenchlorid, Dioxan, 1,2-Dichlorethan, 1,1,2-Trichlorethan, Chloroctan sowie Lösungsmittelgemische wie Pentan/Methylenchlorid, Hexan/Methylenchlorid oder Chlomaphthalin in Frage. Bei Lösungsmittel mit niedrigem Siedepunkt kann durch Anlegen eines ausreichenden Reaktionsdruckes für die Einhaltung der flüssigen Phase gesorgt werden; diese Zusammenhänge sind dem Fachmann bekannt. Die Polymere können durch Nichtlösemittel, wie Methanol, ausgefällt oder umgefällt und dann getrocknet werden.

Geeignete Molybdän- und/oder Wolfram-Carbinkomplexe sind Verbindungen der allgemeinen Formel (III),

$$L_3M{\equiv}CR \qquad \text{(III)}$$

wobei

M = Wolfram oder Molybdän

L = gleich oder verschieden, Halogen, $C_1$-$C_{20}$-Alkoxy, $C_6$-$C_{12}$-Aroxy, sterisch anspruchsvolle $C_3$-$C_{20}$-Alkyl, gegebenenfalls substituierte bzw. benzannellierte Cyclopentadienyle,

R = gleich oder verschieden, $C_1$-$C_{20}$-Alkyl, $C_6$-$C_{12}$-Aryl, Trimethylsilyl,

Bevorzugt ist

M = Wolfram

L = Cl, Br, I, $C_1$-$C_{12}$-Alkoxy, Phenoxy, 2,6-Dimethylphenoxy, 2,6-Diisopropylphenoxy, 2,6-Ditertiärbutyl-phenoxy-tertiär-butoxy

R = Methyl, Ethyl, n-Propyl, i-Propyl, n-Butyl, t-Butyl, s-Butyl, Neopentyl, Trimethylsilyl, Phenyl, substituierte Phenyle, Triphenylsilyl.

Besonders bevorzugte Komplexe sind $Np_3W{\equiv}C^tBu$, $Cl_3(dme)W{\equiv}C^tBu$, $(^tBuO)_3W{\equiv}C^tBu$ und $(^tBuO)_3W{\equiv}C^t$-Bu, wobei Np = *neo*-pentyl = $(CH_3)_3C$-$CH_2$-, dme = 1,2-Dimethoxyethan, $^tBu$ = *tert*-Butyl = $(CH_3)_3C$-.

In der Regel liegen die verwendeten molaren Monomer-Katalysator-Verhältnisse im Bereich von $10^5$ Monomer zu 1 Katalysator und 50 Monomer zu 1 Katalysator, insbesondere $10^2$ Monomer zu 1 Katalysator und $10^4$ Monomer zu 1 Katalysator.

Die Reaktionsdauern liegen üblicherweise im Bereich von wenigen Sekunden bis mehreren Tagen, insbesondere im Bereich von 10 Minuten bis 24 Stunden. Längere Reaktionszeiten sind insbesondere dann sinnvoll, wenn die Reaktionsführung zu einer Ausfällung des Polymers führt (Suspensionspolymerisation).

Bei der Reaktionsführung in Substanz (ohne Lösungsmittel) konnten vorteilhafterweise bei hohen Monomer-Katalysator-Verhältnissen z.B. $\geq$500 lösliche Polymere in hohen Ausbeuten und mit hohen Molekulargewichten erhalten werden.

Die erhältlichen Polymeren zeichnen sich allgemein durch ausreichende Molekulargewichte und Ausbeuten bei guter Löslichkeit der Polymere aus. Dies ist überraschend, da bei Verwendung von klassischen Metathesekatalysatoren häufig unlösliche, vernetzte Produkte mit zudem niedriger Aktivität erhalten werden, die für technische Anwendungen im allgemeinen unbrauchbar sind.

Die im erfindungsgemässen Verfahren erhaltenen Produkte haben eine gute thermische Beständigkeit, Alterungsbeständigkeit, chemische Beständigkeit, gute Gasbarriereeigenschaften, Lösungsmittelbeständigkeit, geringe Wasseraufnahme, hohe Kratzfestigkeit, geringe Lichtdoppelbrechung und hohe Erweichungstemperaturen (Glasübergangstemperaturen $T_g$). Solche (Co)Polymere sind daher u.a. geeignet für: Folien in unverstreckter oder monoaxial oder biaxial verstreckter Form für Verpackungen und als Deckschichten für Polarisationsfolien und Flüssigkristalldisplays, Lackbestandteile, etwa für die Automobilindustrie zur Kratzfestausrüstung von Oberflächen; Fasern, etwa für Lichtwellenleiter; optische Linsen und Prismen; Schläuche, Rohre, Stangen und Trägerplatten; Deckscheiben für Solarzellen; Kondensator-Dielektrikum. Solche technischen Artikel werden üblicherweise durch Spritzguss oder Extrusion hergestellt. Die so eingesetzten (Co)Polymere sind amorph oder nur teilkristallin. Weitere Einsatzgebiete sind, gegebenenfalls nach Hydrierung, optische Datenspeicher oder typische Kautschukanwendungen, wie Profile oder Dichtungen. Die (Co)Polymere können allein oder in Abmischung mit anderen Polymeren angewandt werden. Insbesondere eignen sich zur Abmischung Polystyrol, Polyethylen, Polypropylen, oder Kautschuke, wie BR, NBR, HNBR, IIR, EP(D)M, CR, FKM.

**Beispiele**

**Beispiel 1:** Synthese von $Np_3W{\equiv}C^tBu$

a) Vorstufe: $Me_3Si(OMe)$

In einem 500 ml Schlenk-Dreihalskolben mit Tropftrichter, Rückflusskühler und Überdruck-ventil wurden 200 ml frisch destilliertes Anilin vorgelegt. Anschließend wurden unter starkem Rühren 54 ml Trimethylchlorsilan langsam zugetropft und weiterhin 15 Minuten gerührt. Nun wurden 18 g trockener Methanol zugegeben und anschließend 30 Minuten unter Rückfluss erhitzt. Der Rückflusskühler wurde durch eine Destillationsbrücke ersetzt und das Produkt $Me_3Si(OMe)$ abdestilliert (55-58°C). Zum Destillat wurden Na-Späne zugegeben und man ließ den Kolben über Nacht stehen. (Überdruckventil anschließen, da Gasentwicklung). Am nächsten Tag wurde das Produkt abdekantiert und über eine 30 cm Vigreuxkolonne destilliert (Sdp.: 57°C). $^1H$-NMR ($CDCl_3$),$\delta$ (ppm): 3.33 (s, 3 H, O-C$\underline{H}_3$), 0.03 (s, 9 H, Si(C$\underline{H}_3$)$_3$)

Reaktionsgleichung:

$$Me_3SiNHSiMe_3 + 2\ MeOH \rightarrow 2\ Me_3SiOMe + NH_3 \uparrow$$

b) Vorstufe: Neopentylchlorid (NpCl)

Reinigung des DMF (Dimethylformamid):

1420 ml DMF, 340 ml Toluol und 70 ml Wasser wurden in einen 2000 ml Kolben gefüllt und über eine 30 cm Vigreuxkolonne destilliert. Vorlauf ca 500 ml. Die Hauptfraktion ging bei einer Siedetemperatur von 149-152°C über. Das so vorgereinigte DMF ließ man 48 Stunden über Molekularsieb 4 Å (im Vakuum bei $10^{-3}$ mbar 1 Stunde bei 100°C ausgeheizt) stehen.

Synthese von Chlormethylen-Dimethylammonium-Chlorid nach D.R. Hepburn, H.R. Hudson, *J Chem. Soc. Perkin I* **1976,** 754

In einem 2000 ml Schlenk-Dreihalskolben wurden 1000 ml trockenes DMF vorgelegt und mit einem Isopropanol/Trockeneis-Gemisch auf -5 bis 0°C abgekühlt. Anschließend wurden portionsweise 406,0 g $PCl_5$ langsam über eine PVC-Schlauchverbindung zugegeben (Temperatur darf 0°C nicht übersteigen!). Die Lösung färbte sich zunächst gelb, dann orange und das Salz $[Me_2NCHCl]Cl$ begann auszufallen). Nach Ende der $PCl_5$-Zugabe ließ man auf Raumtemperatur erwärmen. Die entstandene, braune Suspension wurde über Nacht bei -30°C zur vollständige Kristallisation des Salzes aufbewahrt. Am nächsten Tag wurde das entstandene Salz unter Argon mit einer 21-Fritte abfiltriert (das Salz ist feuchtigkeitsempfindlich). Man wusch das Salz mit 500 ml DMF (bis DMF farblos). Nachwaschen mit 3 x 100 ml Ether. Das weiße Salz wurde übernacht im Vakuum trocknet.
Ausbeute: 246,45 g (1,925 mol) weiße Kristalle = 98,8 % bzgl. 1.95 Mol Salz (Lit. 89 %)

Reaktionsgleichung:

$$Me_2NCHO + PCl_5 \xrightarrow{DMF} [Me_2NCHCl]^+Cl^- + POCl_3$$

Umsetzung von $[Me_2NCHCl]Cl$ mit Neopentylalkohol nach D.R. Hepburn, H.R. Hudson, *J. Chem. Soc. Perkin I* **1976,** 754

246.45 g $[Me_2NCHCl]Cl$ (1.925 mmol) wurden in einen 2000 ml Dreihalskolben gegeben, in 1000 ml DMF suspendiert und mit einem Isopropanol/Trockeneis-Gemisch auf 0°C gekühlt. 141.41 g Neopentylalkohol (1.604 mol) wurden in 50 ml DMF in einem Tropftrichter gelöst und bei 0°C zugetropft. Die Lösung erwärmte sich, das Salz löste sich auf und Gasentwicklung trat ein (HCl!). Zwischen Überdruckventil und Kolben kann eine Isopropanol/Trockeneis-Kühlfalle geschaltet werden, deren Inhalt vor dem Destillieren in den Kolben gegeben wird (HCl reißt NpCl etc. mit sich). Dann erhitzte man die Lösung und ließ 6 h unter Rückfluss sieden. Die Lösung färbte sich schwarz. Nach Abkühlen der Lösung auf Raumtemperatur gab man 750 ml Wasser zu. In einem großen Schütteltrichter trennte man die organische Phase ab und wusch sie 3 x mit 100 ml Wasser. (Rohprodukt: Gelbe Flüssigkeit). Man trocknete das Produkt über $K_2CO_3$ (3 h rühren). (Ausbeute Rohprodukt: 147.5 g = 86.3 %, Lit. 73 % vor Destillation). Destillation über VigreuxKolonne: Sdp.: 83-84°C, Reinheit nach GC: 96.7 %. Ausbeute nach Destillation: 119.28 g (1.119 mol) = 69.79 % farblose Flüssigkeit. $^1$H-NMR ($CDCl_3$),δ (ppm) 3.29 (s, 2 H, $CH_2$), 0.98 (s, 9 H, Me).

Reaktionsgleichung:

$$NpOH + [Me_2NCHCl]^+Cl^- \xrightarrow{DMF} NpCl + Me_2NCHO + HCl\uparrow$$

c) Vorstufe: NpMgCl

30.2 g Magnesiumspähne wurden in einen 1000 ml Schlenk-Dreihalskolben eingewogen und im Vakuum ca. 1-2 Stunden auf 150°C erhitzt (Heizpilz Stufe 1). Anschließend im Vakuum abkühlen lassen. Den Reaktionskolben mit Rückflusskühler (Eiskühlung des Kühlwassers) und Tropftrichter ausstatten und in ein Ultraschallbad stellen. Die Magnesiumspähne wurden nun mit 100 ml trockenem Ether überschichtet (Etheroberfläche ca 1 cm über den Mg-Spähnen) und im laufenden Ultraschallbad ca 2 Stunden stehen lassen (Grignard springt dann besser an! Blanke Oberfläche, warme Etherlösung). Anschließend wurden 50 ml NpCl zugegeben. Die restlichen 88.7 ml NpCl wurden mit 100 ml Ether in den Tropftrichter gefüllt. Die Reaktion wird durch Zugabe von ca 1 ml 1,2-Dibromethan gestartet. Nach dem Anspringen des Grignards wird die restliche Ether/NpCl-Lösung langsam zugetropft (Dauer: 2-3 Stunden). Die Reaktionslösung bleibt 2 Tage im Ultraschallbad stehen. Dann wird das Ultraschall ausgestellt und die Grignardlösung auf Raumtemperatur abgekühlt. Die Molarität der Grignardlösung wird durch komplexometrische Titation bestimmt:
Durchschnittlicher Verbrauch an EDTA: 36.83 ml => Lösung ist 3.683 molar. Gesamtmenge der Lösung: ca 330 ml, => ca. 90 % Ausbeute.

Reaktionsgleichung:

$$\mathrm{NpCl} + \mathrm{Mg} \xrightarrow{\text{Ether}} \mathrm{NpMgCl}$$

d) Vorstufe: $LiO^tBu$

2.37 ml (1.853 g, 25 mmol) *tert*-Butanol wurden bei 0°C in 10 ml trockenem Ether vorgelegt und mit 15.62 ml n-Butyl-Lithium (1.6 M in Hexan, 25 mmol) versetzt.
Anschließend lässt man 1 h rühren. Danach entfernt man das Lösungsmittel im Vakuum und sublimiert den Rückstand bei 120°C im Hochvakuum (ca $10^{-4}$ mbar).
Ausbeute: 90 %, weißer Feststoff.
$^{13}$C-NMR δ [ppm] = 66.6 (**C**$Me_3$), 35.3 (C**$Me_3$**).

Reaktionsgleichung:

$$^{t}\mathrm{BuOH} + \mathrm{BuLi} \xrightarrow{\text{Ether}} {}^{t}\mathrm{BuOLi} + \mathrm{BuH}\uparrow$$

e) Vorstufe: $Cl_3W(OMe)_3$ nach L.B. Handy, K.G. Sharp, F.E. Brinckman, *Inorg. Chem*. **1972**, *11*, 523

104 ml $Me_3SiOMe$ wurden in einem 500 ml Schlenkkolben unter Argon vorgelegt und auf 0°C gekühlt. Über eine PVC-Schlauchverbindung wurden nun 100 g $WCl_6$ in kleinen Portionen zugegeben (Zeitbedarf ca. 10 h!). Bei der Zugabe des $WCl_6$ verfärbt sich die Lösung nach rotbraun um nach einigen Minuten wieder gelb zu werden. Erneute Zugabe erfolgt erst nach Erscheinen dieser Gelbfärbung. Über Nacht rühren und dabei auf Raumtemperatur erwärmen lassen. Reaktionsdauer 24h.

Das gebildete $Me_3SiCl$ wird abgezogen, der Rückstand in ca 100 ml $CH_2Cl_2$ gelöst und abfiltriert (mit $CH_2Cl_2$ nachwaschen, hellblauer Rückstand). Das Lösungsmittel im Vakuum einengen und den braunen Rückstand im Vakuum trocknen.
Ausbeute: 88 % $Cl_3W(OMe)_3$ (= Literaturausbeute).
$^{1}$H-NMR ($CDCl_3$),δ (ppm): 5.46 (s, 6 H, OC**$H_3$**, cis, cis), 5.40 (s, 3 H, OC**$H_3$**, cis, trans),
$^{13}$C-NMR ($CDCl_3$),δ (ppm): 71.5, 70.5, 67.6 (O**Me**, cis, cis und cis, trans).

Reaktionsgleichung:

$$3\ Me_3SiOMe + WCl_6 \rightarrow Cl_3W(OMe)_3 + 3\ Me_3SiCl$$

f) Endstufe: $Np_3W{\equiv}C^tBu$

194.7 ml NpMgCl (0.717 mol) 6 Molteile der Grignardlösung (bezogen auf 1 Teil $Cl_3W(OMe)_3$) wurden in einem 1000 ml Schlenkkolben unter Argon mit 523 ml trockenem Ether zu einer 1 molaren Lösung verdünnt. Nun wurden bei 0°C ca. 45.81 g $Cl_3W(OMe)_3$ über eine Schlauchverbindung in kleinen Portionen zugegeben (Zeitbedarf ca 4-8 h). Die weitere Zugabe erfolgt erst nach Beendigung der Gasentwicklung. Anschließend lässt man das Reaktionsgemisch über Nacht rühren und weitere 1-2 Tage bei Raumtemperatur stehen. Die gebildete Suspension wird über eine große 2 1-Fritte und eine kleinere Fritte abfiltriert. Das Filtrat in einen 1000 ml Kolben auffangen. Den Rückstand beim Filtrieren absetzen lassen. Der abgefangene Rückstand wird unter Aufrühren mit Pentan versetzt und solange mit Pentan nachgewaschen bis die Waschlösung farblos ist. Es ist darauf zu achten, dass der Rückstand vollständig abgetrennt wird, da dieser bei der anschließenden Feststoffdestillation stört. Die gesammelten Pentanextrakte wurden im Vakuum eingeengt, anschließend in die Feststoffdestille umgefüllt und über Nacht bei Raumtemperatur (Wasserbad) im Hochvakuum weiter eingeengt.
Das zur Feststoffdestillation benötigte Vakuum sollte besser als $10^{-3}$ mbar sein. Die Temperatur des Thermostaten wird langsam auf 80°C bis maximal 100°C hochgeregelt. Bei höheren Temperaturen erfolgt Zersetzung. Die Destillation setzt bei ca 60°C ein. Die Vorlage wird mit flüssig-$N_2$ gekühlt. Als Produkt geht ein orangefarbenes Öl über, das einen gelben Feststoff ergibt.
Ausbeute: 39.74 g (= 71.4%). Die Charakterisierung erfolgt durch NMR-Spektroskopie.
$^1$H-NMR, ($CDCl_3$), δ [ppm] = 1.39 (s, 9H, $W{\equiv}CC\underline{\mathbf{Me}}_3$), 1,04 (s, 27H, $(-CH_2C\underline{\mathbf{Me_3}})_3$), 0,89 (s, 6H, $(-C\underline{\mathbf{H}}_2CMe_3)_3$).,
$^{13}$C-NMR, ($CDCl_3$), δ [ppm] = 315.6 ($W{\equiv}\underline{\mathbf{C}}CMe_3$), 103.0 ($\underline{\mathbf{C}}H_2CCMe_3$), 52.5 ($C\underline{\mathbf{C}}Me_3$), 38.4 ($CH_2\underline{\mathbf{C}}Me_3$), 34.2 ($CH_2C\underline{\mathbf{Me}}_3$), 32.28 ($CC\underline{\mathbf{Me}}_3$).

Reaktionsgleichung:

$$Cl_3W(OMe)_3 + 6\ NpMgCl \xrightarrow{\text{Ether}} Np_3W{\equiv}C^tBu + 2\ NpH\uparrow + 3\ MgCl_2 + 3\ Mg(OMe)Cl$$

**<u>Beispiel 2</u>**: Synthese von $Cl_3(dme)W{\equiv}C^tBu$

1.9 g (4.08 mmol, 1 Molteil) $Np_3W{\equiv}CCMe_3$ aus Beispiel 1 werden unter Argon, bei 0°C in 50 ml Pentan gelöst und mit 1.3 ml (12.24 mmol, drei Molteile) 1,2-Dimethoxyethan (dme) versetzt.
Dann wird im Argonstrom HCl-Gas direkt aus der Bombe in die Pentanlösung eingeleitet <u>(nur ca 30 Sekunden !!!)</u>. Es beginnt sofort ein violetter Niederschlag auszufallen. Man lässt für ca 15 min Argon durch die Suspension blubbern, um überschüssiges HCl aus der Lösung zu entfernen. Die überstehende Lösung wird abdekantiert und der Niederschlag 3 mal mit 20 ml Pentan gewaschen. Der Carbinkomplex wird am Hochvakuum getrocknet.
<u>Ausbeute:</u> 1.41 g (77%). Die Charakterisierung erfolgt durch NMR-Spektroskopie.
$^1$H-NMR, δ [ppm] = 4.43 (s, 3H, $O-C\underline{\mathbf{H}}_3$), 4.09 (t, 2H, $-C\underline{\mathbf{H}}_2-O$), 3.81 (t, 2H, $-C\underline{\mathbf{H}}_2-O$), 3.67 (s, 3H, $O-C\underline{\mathbf{H}}_3$), 1.25 (s, 9H, $-C(C\underline{\mathbf{H}}_3)_2$).
$^{13}$C-NMR, δ [ppm] = 336.4 ($W{\equiv}\underline{\mathbf{C}}-C$), 78.5 ($-\underline{\mathbf{C}}H_2-O$), 76,6 ($O-\underline{\mathbf{C}}H_3$), 69,7 ($-\underline{\mathbf{C}}H_2-O$), 59,6 ($O-\underline{\mathbf{C}}H_3$), 47.7 ($=C-\underline{\mathbf{C}}(CH_3)_2$), 33.3 ($\equiv C-C(\underline{\mathbf{C}}H_3)_3$).

Reaktionsgleichung:

$$Np_3W\equiv C^tBu \;+\; 3\,HCl \;+\; dme \;\xrightarrow{\text{Pentan}}\; Cl_3(dme)W\equiv C^tBu \;+\; 3\,NpH\uparrow$$

**Beispiel 3**: Synthese von $(^tBuO)_3W\equiv C^tBu$

759 mg (9.48 mmol, 3 Molteile) $LiO^tBu$ werden bei 0°C in trockenem Ether gelöst. Anschließend werden 1420 mg (3.16 mmol, 1 Molteil) $Cl_3(dme)W\equiv CCMe_3$ aus Beispiel 2 in kleinen Portionen zugegeben (ca 30 min) und im weiteren 1 h gerührt (0°C). Man lässt das entstandene LiCl absetzen und pipettiert die überstehende Lösung ab. Das Lösungsmittel wird im Hochvakuum abgezogen. Der Rückstand wird mit 50 ml Pentan versetzt und über Nacht bei -80°C aufbewahrt, damit sich LiCl-Reste absetzen. Die überstehende Lösung wird abpipettiert und das Lösungsmittel im Vakuum abgezogen. Der beigebraune Carbinkomplex wird im Hochvakuum getrocknet. Ausbeute: 1249 mg $(^tBuO)_3W\equiv CCMe_3$ (83.7 %).
$^1$H-NMR ($CDCl_3$),δ [ppm] 1.41 (s, 27 H, $(OC(C\underline{\mathbf{H}}_3)_3)$), 1.21 (s, 9 H, $W\equiv CC(C\underline{\mathbf{H}}_3)_3)$).
$^{13}$C-NMR ($CDCl_3$),δ [ppm] 271.5 ($W\equiv\underline{\mathbf{C}}C$), 78.7 ($O\underline{\mathbf{C}}Me_3$), 49.6 ($C\underline{\mathbf{C}}Me_3$), 33.9 ($CC\underline{\mathbf{Me_3}}$), 32.4 ($OC\underline{\mathbf{Me_3}}$).

Reaktionsgleichung:

$$Cl_3(dme)W\equiv C^tBu + 3\ {}^tBuOLi \rightarrow ({}^tBuO)_3W\equiv C^tBu + 3\ LiCl + dme$$

**Beispiel 4**: Polymerisation von 5-Cyano-2-Norbornen mit $(^tBuO)_3W\equiv C^tBu$ als Katalysator in Lösung

430 mg (3.6 mmol) 5-Cyano-2-Norbornen wurden in einem vorher inertisierten 200 ml Schlenkrohr in 10 ml $CH_2Cl_2$ und 5 ml Hexan gelöst. Bei Kinetiken wurden 0.5 ml Decan als internen Standard für die gaschromatographische Umsatzbestimmung zugefügt. 17 mg (0.036 mmol) $(^tBuO)_3W\equiv C^tBu$ wurden in ein 50 ml Schlenkrohr eingewogen und in 1 ml $CH_2Cl_2$ gelöst. Das molare Verhältnis 5-Cyano-2-Norbornen : $(^tBuO)_3W\equiv C^tBu$ beträgt 100 : 1. Das Schlenkrohr wird zur Temperaturkonstanz in ein Wasserbad (25°C) gestellt. Die Polymerisation wird durch Zugabe der Katalysatorlösung zur Monomerlösung gestartet. Nach 24 Stunden wird die Reaktion mit 0.1 ml Benzaldehyd abgebrochen, das Polymer mit 100 ml Methanol gefällt und filtriert. Zur Reinigung wird das Polymere in 10 ml $CH_2Cl_2$ gelöst und erneut in 100 ml Methanol gefällt, filtriert und im Vakuum ($10^{-3}$ mbar) bei 25°C bis zur Gewichtskonstanz getrocknet. Die Ausbeute an Polymer beträgt 375 mg = 87.2 %. Das entstandene Poly(5-Cyano-2-Norbornen) hatte ein Molekulargewicht von $\overline{M}_w$ = 13 700 g/mol, die Polydispersität lag bei D = 3.18.

**Beispiel 5:** Polymerisation von 5-Cyano-2-Norbornen mit $(^tBuO)_3W\equiv C^tBu$ als Katalysator in Substanz:

In ein vorher inertisiertes 150 ml Schlenkrohr wurden 22 mg (46.6 μmol) (tBuO)3W CtBu eingewogen. Dann wurden 2775 mg 5-Cyano-2-Norbornen (23.3 mmol, molares Verhältnis 5-Cyano-2-Norbornen : $(^tBuO)_3W\equiv C^tBu$ = 500 : 1) hinzugefügt und das Gemisch gut durchmischt. Das Schlenkrohr wird zur Temperaturkonstanz in ein Wasserbad (25°C) gestellt. Die Polymerisation erfolgt sofort, die Reaktionsmischung wird warm und viskos. Nach 60 minütiger Reaktionszeit wird das entstandene Polymere in 20 ml $CH_2Cl_2$ gelöst und die Reaktion durch Zugabe von 0.1 ml Benzaldehyd abgebrochen. Das entstandene Poly(5-Cyano-2-Norbornen) wird in 100 ml MeOH gefällt. Es resultiert ein weißes Polymer. Ausbeute 2636 mg = 95 % Poly(5-Cyano-2-Norbornen). Das entstandene Poly(5-Cyano-2-Norbornen) hatte ein Molekulargewicht von $\overline{M}_w$ = 583 400 g/mol, die Polydispersität lag bei D = 2.32.

**Beispiel 6**: Polymerisation von 5-Cyano-2-Norbornen mit $Cl_3(dme)W\equiv C^tBu$ als Katalysator in Lösung:

Analog zu Beispiel 4 wurde 5-Cyano-2-Norbornen mit dem Wolfram-Carbin-Komplex $Cl_3(dme)W\equiv C^tBu$ in 10 ml $CH_2Cl_2$ einem molaren Verhältnis 5-Cyano-2-Norbornen : $Cl_3(dme)W\equiv C^tBu$ = 75 : 1 umgesetzt. Dazu wurden 50 mg (111 μmol) $Cl_3(dme)W\equiv C^tBu$ eingewogen. Poly(5-Cyano-2-Norbornen) wurde in 37 %-iger Ausbeute erhalten. Das

entstandene Poly(5-Cyano-2-Norbornen) hatte ein Molekulargewicht von $\overline{M}_w$ = 7660 g/mol, die Polydispersität lag bei D = 1.57.

**Beispiel 7**: Polymerisation von 5-Cyano-2-Norbornen mit $Cl_3(dme)W{\equiv}C^tBu$ als Katalysator in Substanz:

Analog zu Beispiel 5 wurde 5-Cyano-2-Norbornen mit dem Wolfram-Carbin-Komplex $Cl_3(dme)W{\equiv}C^tBu$ in Substanz umgesetzt. Dazu wurden 23 mg (51 μmol) $Cl_3(dme)W{\equiv}C^tBu$ eingewogen. Bei einem molaren Monomer-Katalysator-Verhältnis 5-Cyano-2-Norbornen : $Cl_3(dme)W{\equiv}C^tBu$ = 500 : 1 konnte Poly(5-Cyano-2-Norbornen) in 18.7 %-iger Ausbeute erhalten werden. Dieses Polymere hatte ein $\overline{M}_w$ von 17630 g/mol und eine Molekulargewichtsverteilung von D = 1.2.

**Beispiel 8**: Polymerisation von 5-Cyano-2-Norbornen mit $Np_3W{\equiv}C^tBu$ als Katalysator in Lösung:

Analog zu Beispiel 4 wurde 5-Cyano-2-Norbornen mit dem Wolfram-Carbin-Komplexen $Np_3W{\equiv}C^tBu$ als Katalysator in 10 ml Hexan als Lösungsmittel umgesetzt. Dazu wurden 78 mg (167.8 μmol) $Np_3W{\equiv}C^tBu$ eingewogen. Es konnte Poly(5-Cyano-2-Norbornen) in 31.1 %-iger Ausbeute erhalten werden. Dieses Polymere hatte ein Molekulargewicht von $\overline{M}_w$ = 214 600 g/mol. Die Polydispersität dieses Polymeren lag bei D = 6.7.

**Beispiel 9** (Vergleich):

Polymerisation von Cyclopenten mit $(^tBuO)_3$ $W{\equiv}C^tBu$ als Katalysator in Lösung:

Die Polymerisation wurde analog zu Beispiel 4 durchgeführt. Anstelle von 5-Cyano-2-Norbornen wurden 202 mg (2.96 mmol) Cyclopenten mit einem Monomer-Katalysator-Verhältnis von Cyclopenten : $(^tBuO)_3$ $W{\equiv}C^tBu$ = 100 : 1 eingewogen. Als Lösungsmittel wurde Decan verwendet. Die Ausbeute an Poly(Cyclopenten) betrug 69.4 %. Das entstandene Poly(Cyclopenten) hatte ein Molekulargewicht von $\overline{M}_w$ = 100 300 g/mol, die Polydispersität lag bei D = 2.91.

**Beispiel 10** (Vergleich):

Polymerisation von Dicyclopentadien mit $(^tBuO)_3$ $W{\equiv}C^tBu$ als Katalysator in Lösung:

Die Polymerisation wurde analog zu Beispiel 4 durchgeführt. Anstelle von 5-Cyano-2-Norbornen wurden 616 ma (4.66 μmol) Dicyclopentadien mit einem Monomer-Katalysator-Verhältnis von Dicyclopentadien : $(^tBuO)_3W{\equiv}C^tBu$ = 100 : 1 eingewogen. Als Lösungsmittel wurde das Gemisch aus 10 ml $CH_2Cl_2$ und 5 ml Hexan verwendet. Die Ausbeute an Poly(Dicyclopentadien) betrug 99 %. Das entstandene Poly(Dicyclopentadien) hatte ein Molekulargewicht von $\overline{M}_w$ = 158 900 g/mol, die Polydispersität lag bei D = 3.5.

**Beispiel 11:** Synthese des neuen 5-Cyano-5-Octyl-2-Norbornen

Zur Darstellung eines octylsubstituierten 5-Cyano-2-Norbornens wurden 5.9 ml (5.84 g = 49.4 mmol) 5-Cyano-2-Norbornen in einem vorher inertisierten 200 ml Schlenkrohr in 60 ml THF gelöst und auf -78°C abgekühlt (Trockeneis Isopropanol). Dann wurden unter Argon 6.02 g (56.2 mmol) Lithiumdiisopropylamid (LDA) portionsweise zugegeben. Diese Reaktionslösung ließ man eine Stunde bei -78°C rühren. Danach wurden 13 ml (11.24 g = 75.6 mmol) Octylchlorid langsam unter Argon zugegeben und man ließ die Reaktionslösung nochmals eine Stunde bei -78°C rühren. Dann ließ man den Ansatz auf Raumtemperatur erwärmen, das entstandene LiCl absetzen und man dekantierte die überstehende Lösung ab. Die orangefarbene Lösung wurde im Vakuum ($10^{-3}$ mbar) eingeengt und anschließend im Vakuum destilliert: gelbliche Flüssigkeit: Sdp.($10^{-3}$ mbar): 120-125°C, Ausbeute: 6.4 g = 56.1 % (bzgl. 49.4 mmol 5-Cyano-2-Norbornen). Isomerenverteilung (gaschromatographisch bestimmt): 4.95 % exo und 92.6 % endo. Charakterisierung: Dichte: 0.88 g/ml, (durch wiegen von 1 ml 5-Cyano-5-Octyl-2-Norbornen bestimmt).
$^1$H-NMR (250 MHz, $CDCl_3$) δ = 6.23 (H-2 und 3), 2.81 (H-4), 2.88 (H-1), 1.61 (H-9). 1.41 (H-10 bis 15), 1.24 (H-6 und 7), 0.81 (H-16) ppm.
$^{13}$C-NMR (63 MHz, $CDCl_3$) δ= 138.6 (C-2), 135.1 (C-3), 124.8 (C-8), 49.7 (C-7), 48.6 (C-4), 42.3 (C-1), 42.2 (C-5), 40.3 (C-6), 39.3 (C-10), 31.6 (C-14), 29.4 (C-13), 29.3 (C-12), 29.1 (C-11), 26.2 (C-9), 22.3 (C-15), 13.8 (C-16) ppm.

**Beispiel 12:** Polymerisation von 5-Cyano-5-octyl-2-norbornen mit $(^tBuO)_3W{\equiv}C^tBu$ als Katalysator in Lösung:

Die Polymerisation wurde analog zu Beispiel 4 durchgeführt. Anstelle von 5-Cyano-2-norbornen wurden 1500

mg (6.0 mmol) 5-Cyano-5-octyl-2-norbornen mit einem Monomer-Katalysator-Verhältnis von 5-Cyano-5-octyl-2-norbornen : $(^tBuO)_3$ W≡$C^tBu$ = 500 : 1 eingewogen. Als Lösungsmittel wurden 5 ml $CH_2Cl_2$ verwendet. Die Ausbeute an Poly(5-Cyano-5-octyl-2-norbornen) betrug 76 %. Das entstandene Poly(5-Cyano-5-octyl-2-norbornen) hatte ein Molekulargewicht von $\overline{M}_w$ = 559 700 g/mol, die Polydispersität lag bei D = 3.38.

**Beispiel 13**: Co-Polymerisation von 5-Cyano-2-Norbornen und Dicyclopentadien mit $(^tBuO)_3W≡C^tBu$ als Katalysator (äquimolare Monomermengen)

In ein inertisiertes 200 ml Schlenkrohr wurden 464 mg (3.9 mmol) 5-Cyano-2-Norbornen und 516 mg (3.9 mmol) Dicyclopentadien eingewogen und in 10 ml $CH_2Cl_2$ und 5 ml Hexan (und in 0.5 ml Decan als internen Standard für die gaschromatographische Umsatzbestimmung) gelöst. In ein 10 ml Schlenkrohr wurden 19 mg (39 µmol) $(^tBuO)_3W≡C^tBu$ eingewogen und in 1 ml $CH_2Cl_2$ gelöst. Das molare Verhältnis 5-Cyano-2-Norbornen : Dicyclopentadien : $(^tBuO)_3W≡C^tBu$ beträgt 100 : 100 : 1. Die Copolymerisation wird durch Zugabe der Katalysatorlösung zur Monomerlösung gestartet. Das Schlenkrohr wird zur Temperaturkonstanz in ein Wasserbad (25°C) gestellt. Bei Kinetiken wurden Proben zur gaschromatographischen Umsatzbestimmung entnommen. Nach 24 Stunden Reaktionsdauer wird die Reaktion mit 0.1 ml Benzaldehyd abgebrochen, das Polymere in 100 ml Methanol gefällt, filtriert und im Vakuum ($10^{-3}$ mbar) bei 25°C bis zur Gewichtskonstanz getrocknet. Es resultiert ein weißes Copolymer. Ausbeute 812 mg = 90 % Poly(5-Cyano-2-Norbornen /Dicyclopentadien). Das Molekulargewicht betrug 140 500 g/mol, D = 2.47.

**Beispiel 14:** Co-Polymerisation von 5-Cyano-2-Norbornen und Dicyclopentadien mit $(^tBuO)_3W≡C^tBu$ als Katalysator (äquimolare Monomermengen)

Die Copolymerisation wurde analog zu Beispiel 13 durchgeführt. Es wurden jedoch 20 mg (42.3 µmol) $(^tBuO)_3W≡C^tBu$, 504 mg (4.23 mmol) 5-Cyano-2-Norbornen und 560 mg (4.23 mmol) Dicyclopentadien eingewogen. Die Reaktionsdauer wurde auf 90 Minuten verkürzt. Die Ausbeute an Poly(5-Cyano-2-Norbornen /Dicyclopentadien) betrug 57 %. Das entstandene Poly(5-Cyano-2-Norbornen /Dicyclopentadien) hatte ein Molekulargewicht von $\overline{M}_w$ = 215 000 g/mol, die Polydispersität lag bei D = 2.17.

**Beispiel 15**: Co-Polymerisation von 5-Cyano-2-Norbornen und Dicyclopentadien mit $(^tBuO)_3W≡C^tBu$ als Katalysator (verschiedene Monomerverhältnisse)

Die Reaktion wird analog zu Beispiel 13 durchgeführt. Es wurden jedoch 18 mg (38.1 µmol) (tBuO)3W≡CtBu, 454 mg (3.8 mmol) 5-Cyano-2-Norbornen und 252 mg (1.9 mmol) Dicyclopentadien eingewogen. Das molare Verhältnis 5-Cyano-2-Norbornen : Dicyclopentadien : $(^tBuO)_3W≡C^tBu$ beträgt 100 : 50 : 1. Nach 24 stündiger Reaktionsdauer konnte ein THF-lösliches (GPC-Messung) Copolymeres in 94 %-iger Ausbeute erhalten werden ($\overline{M}_w$ = 349 150 g/mol, D = 2.75).

**Beispiel 16**: Co-Polymerisation von 5-Cyano-2-Norbornen und Dicyclopentadien mit $(^tBuO)_3W≡C^tBu$ als Katalysator (verschiedene Monomerverhältnisse)

Die Reaktion wird analog zu Beispiel 15 durchgeführt. Es wurden jedoch 56 mg (0.4 mmol) Dicyclopentadien eingewogen. Das molare Verhältnis 5-Cyano-2-Norbornen : Dicyclopentadien : $(^tBuO)_3W≡C^tBu$ beträgt 100 : 11 : 1. Nach 24 stündiger Reaktionsdauer konnte ein THF-lösliches (GPC-Messung) Copolymeres in 98 %-iger Ausbeute erhalten werden ($\overline{M}_w$ = 184 220 g/mol, D = 2.71).

**Beispiel 17**: Co-Polymerisation von 5-Cyano-2-Norbornen und Dicyclopentadien mit $(^tBuO)_3W≡C^tBu$ als Katalysator (verschiedene Monomerverhältnisse)

Die Reaktion wird analog zu Beispiel 13 durchgeführt. Es wurden jedoch 16 mg (33.9 µmol) $(^tBuO)_3W≡C^tBu$, 404 mg (3.4 mmol) 5-Cyano-2-Norbornen und 895 mg (6.8 mmol) Dicyclopentadien eingewogen. Das molare Verhältnis 5-Cyano-2-Norbornen : Dicyclopentadien : $(^tBuO)_3W≡C^tBu$ beträgt 100 : 200 : 1. Nach 24 stündiger Reaktionsdauer konnte ein THF-lösliches (GPC-Messung) Copolymeres erhalten werden ($\overline{M}_w$ = 389 400 g/mol, D = 2.96).

**Beispiel 18:** Co-Polymerisation von 5-Cyano-2-Norbornen und Dicyclopentadien mit $(^tBuO)_3W≡C^tBu$ als Katalysator (verschiedene Monomerverhältnisse)

Die Reaktion wurde analog zu Beispiel 13 durchgeführt. Es wurden jedoch 7 mg (14.8 µmol) $(^tBuO)_3W≡C^tBu$.

392 mg (3.0 mmol) 5-Cyano-2-Norbornen und 353 mg (3.0 mmol) Dicyclopentadien eingewogen. Es wurde die Reaktionsdauer auf 5 Minuten verkürzt. Das molare Verhältnis 5-Cyano-2-Norbornen : Dicyclopentadien : $(^tBuO)_3W{\equiv}C^tBu$ betrug 200 : 200 : 1. Die Ausbeute lag bei 15 %, das Molekulargewicht bei $\bar{M}_w$ = 182 300 g/mol, die Polydispersität bei D = 1.6.

**Beispiel 19:** Co-Polymerisation von 5-Cyano-2-Norbornen und Dicyclopentadien mit $(^tBuO)_3W{\equiv}C^tBu$ als Katalysator (verschiedene Monomerverhältnisse)

Die Reaktion wurde analog zu Beispiel 13 durchgeführt. Es wurden jedoch 11 mg (22.1 µmol) $(^tBuO)_3W{\equiv}C^tBu$, 793 mg (6.63 mmol) 5-Cyano-2-Norbornen und 294 mg (2.21 mmol) Dicyclopentadien eingewogen. Das entspricht einem molaren Verhältnis von 5-Cyano-2-Norbornen : Dicyclopentadien : $(^tBuO)_3W{\equiv}C^tBu$ = 300 : 100 : 1. Die Ausbeute lag bei 20 %, das Molekulargewicht bei $\bar{M}_w$ = 122 800 g/mol, die Polydispersität bei D = 1.6.

**Beispiel 20**: Co-Polymerisation von 5-Cyano-2-Norbornen und Cyclopenten mit $(^tBuO)_3W{\equiv}C^tBu$ als Katalysator (äquimolare Monomennengen)

Die Copolymerisation wurde analog zu Beispiel 13 durchgeführt. Es wurden jedoch 18 mg (38.1 µmol) $(^tBuO)_3W{\equiv}C^tBu$, 455 mg (3.81 mol) 5-Cyano-2-Norbornen und 260 mg (3.81 mmol) Cyclopenten eingewogen. Bei einem molaren Verhältnis von 5-Cyano-2-Norbornen : Cyclopenten : $(^tBuO)_3W{\equiv}C^tBu$ = 100 : 100 : 1 wurde ein Copolymeres in 28 %-iger Ausbeute erhalten. $\bar{M}_w$ = 128 500 g/mol, D = 2.35.

**Beispiel 21:** Co-Polymerisation von 5-Cyano-2-Norbornen und Cyclopenten mit $(^tBuO)_3W{\equiv}C^tBu$ als Katalysator (äquimolare Monomermengen)

Die Copolymerisation wurde analog zu Beispiel 13 durchgeführt. Es wurden jedoch 18 mg (38.1 µmol) $(^tBuO)_3W{\equiv}C^tBu$, 908 mg (7.62 mol) 5-Cyano-2-Norbornen und 520 mg (7.62 mmol) Cyclopenten eingewogen. Bei einem molaren Verhältnis von 5-Cyano-2-Norbornen : Cyclopenten : $(^tBuO)_3W{\equiv}C^tBu$ = 200 : 200 : 1 wurde ein Copolymeres in 21 %-iger Ausbeute erhalten. $\bar{M}_w$ = 216 000 g/mol D = 5.

**Beispiel 22**: Co-Polymerisation von 5-Cyano-2-Norbornen und Cyclopenten mit $(^tBuO)_3W{\equiv}C^tBu$ als Katalysator (äquimolare Monomermengen)

Die Copolymerisation wurde analog zu Beispiel 13 durchgeführt. Es wurden jedoch 2 ml Decan und 2 ml THF als Lösungmittel verwendet. Es konnte ein Copolymer in 34 %-iger Ausbeute mit einem Molekulargewicht von $\bar{M}_w$ = 366 400 g/mol erhalten werden (D = 3.47).

**Beispiel 23:** Co-Polymerisation von 5-Cyano-2-Norbornen und Cyclopenten mit $(^tBuO)_3W{\equiv}C^tBu$ als Katalysator (äquimolare Monomermengen)

Die Copolymerisation wurde analog zu Beispiel 13 durchgeführt. Es wurde jedoch 1 ml $CH_2Cl_2$ als Lösungmittel verwendet. Es konnte ein Copolymer in 55 %-iger Ausbeute mit einem Molekulargewicht von $\bar{M}_w$ = 397 900 g/mol erhalten werden (D = 2.63).

**Beispiel 24:** Co-Polymerisation von 5-Cyano-2-Norbornen und Cyclopenten mit $(^tBuO)_3W{\equiv}C^tBu$ als Katalysator (verschiedene Monomermengen)

Die Copolymerisation wurde analog zu Beispiel 13 durchgeführt. Es wurden jedoch 11 mg (22.1 µmol) $(^tBuO)_3W{\equiv}C^tBu$, 793 mg (6.63 mmol) 5-Cyano-2-Norbornen und 294 mg (2.21 mmol) Cyclopenten eingewogen. Es wurde ein molares Verhältnis von 5-Cyano-2-Norbornen : Cyclopenten : $(^tBuO)_3W{\equiv}C^tBu$ = 300 : 100 : 1 eingesetzt. Als Lösungsmittel wurde 1,2 Dichlorethan ($C_2H_4Cl_2$) verwendet. Es konnte Poly(5-Cyano-2-Norbornen /Cyclopenten) in 64 %-iger Ausbeute erhalten werden. Das Molekulargewicht dieses Copolymeren betrug $\bar{M}_w$ = 484 500 g/mol. Eine DSC-Messung dieses Copolymeren ergab einen Glasübergangsbereich von $T_g$ = 109 °C.

**Beispiel 25:** Synthese von 5-Decyl-2-norbornen ($NBEC_{10}H_{21}$)

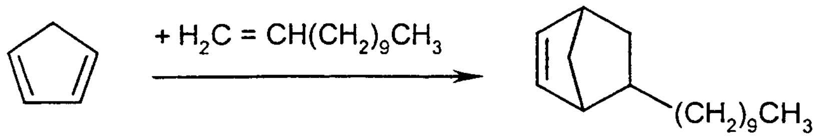


Zur Darstellung des 5-Decyl-2-nornbornens wurde in Abwandlung zur Synthese von T. Sagane und A. Mizuno zunächst Cyclopentadien durch thermisches Cracken von Dicyclopentadien frisch hergestellt (Sdp.: 40°C). (nach Hünig, Märkl, Sauer, *"Integriertes Organisches Praktikum"* **1979**, 78-79).

Dann wurden in einem 200 ml Handautoklaven mit Glaseinsatz 85 ml 1-Dodecen (0,66 mol) und 17 ml Cyclopentadien (0,33 mol) eingefüllt, der Autoklav verschlossen und auf 200°C erhitzt. Dabei stieg der Druck auf 10 bar an. Nach 30 Minuten wurde die Heizung abgeschaltet und man ließ den Autoklaven auf Raumtemperatur abkühlen. Die gelbliche Flüssigkeit wurde zweimal im Vakuum destilliert. Sdp. ($10^{-3}$- mbar): 120°C, Ausbeute: 33 g = 42,7 %; farblose Flüssigkeit, exo-5-Decyl-2-norbornen: 4,5 %; endo-5-Decyl-2-norbornen: 91,5 %.

Die NMR-Daten entsprechen den Literaturangaben von T. Sagane und A. Mizuno. (T. Sagane, A. Mizuno, Makromol. Chem. 1993, 194, 37-52)

**Beispiel 26**: Co-ROMP von 5-Cyano-2-norbornen und 5-Decyl-2-norbornen mit $(^tBuO)_3W{\equiv}C^tBu$ als Katalysator in Lösung

844 mg (3,6 mmol) 5-Decyl-2-norbornen und 429 mg (3,6 mmol) 5-Cyano-2-norbomen werden in einem vorher inertisierten 200 ml Schlenkrohr in 10 ml $CH_2Cl_2$ und 5 ml Hexan gelöst. Zur gaschromatographischen Umsatzbestimmung werden 0,5 ml Decan als internen Standard zugefügt. 17 mg (0,036 mmol) $(^tBuO)_3W{\equiv}C^tBu$ werden in ein 50 ml Schlenkrohr eingewogen und in 1 ml $CH_2Cl_2$ gelöst. Das molare Verhältnis 5-Cyano-2-norbornen : 5-Decyl-2-norbornen : $(^tBuO)_3W{\equiv}C^tBu$ = 100 : 100 : 1. Das Schlenkrohr wird zur Temperaturkonstanz in ein Wasserbad (25°) gestellt. Die Polymerisation wird durch Zugabe der Katalysatorlösung zur Monomerlösung gestartet. Nach 24 Stunden wird die Reaktion mit 0,1 ml Benzaldehyd abgebrochen, das Polymer mit 100 ml Methanol gefällt und filtriert. Zur Reinigung wird das Polymere in 10 ml $CH_2Cl_2$ gelöst und erneut in 100 ml Methanol gefällt, filtriert und im Vakuum ($10^{-3}$ mbar) bei 25°C bis zur Gewichtskonstanz getrocknet. Die Ausbeute an Polymer beträgt 1040 mg = 82 %. Das entstanden Copolymer hatte ein Molekulargewicht von $\overline{M}_w$ = 182 800 g $mol^{-1}$, die Dispersität lag bei D = 2,66. Bei Versuchen mit hohem 5-Decyl-2-norbornen-Anteil im Ausgangsgemisch erhielt man Copolymere mit Kautschuk-artigen Eigenschaften (tiefe $T_g$). Wurde 5-Cyano-2-norbornen im Überschuss eingesetzt, so erhielt man faserige Copolymere mit hoher $T_g$.

**Patentansprüche**

1. Verfahren zur Polymerisation von Cycloalkenen mit polaren Substituenten ausgewählt aus der Gruppe von -CN, Halogen, $-CF_3$, $-C_2F_5$ bis $C_{20}$-$F_{41}$, -N($C_1$-$C_{30}$-Alkyl)$_2$, $C_1$-$C_{20}$-Alkylen-$COOC_1$-$C_{20}$-alkyl, Hydroxy, Hydroxyalkyl gegebenenfalls in Anwesenheit einer oder mehreren weiteren ungesättigten Verbindungen, **dadurch gekennzeichnet, dass** in Gegenwart einer oder mehrerer Wolfram-Carbin-Komplexe und/oder Molybdän-Carbin-Komplexe polymerisiert wird, wobei das Zentralmetall formal in der Oxidationsstufe + VI vorliegt.

2. Verfahren gemäss Anspruch 1, **dadurch gekennzeichnet, dass** in Abwesenheit eines Lösungsmittels polymerisiert wird.

**Claims**

1. Process for the polymerization of cycloalkenes having polar substituents selected from the group consisting of -CN, halogen, $-CF_3$, $-C_2F_5$ to $C_{20}$-$F_{41}$, -N($C_1$-$C_{30}$-alkyl)$_2$, $C_1$-$C_{20}$-alkylene-$COOC_1$-$C_{20}$-alkyl, hydroxyl and hydroxyalkyl, optionally in the presence of one or more further unsaturated compounds, **characterized in that** the polymerization is carried out in the presence of one or more tungsten-carbyne complexes and/or molybdenum-carbyne complexes, the central metal being formally in the +VI oxidation state.

**2.** Process according to Claim 1, **characterized in that** the polymerization is carried out in the absence of a solvent.

## Revendications

**1.** Procédé pour la polymérisation de cycloalcènes à substituants polaires choisis parmi les groupes -CN, les halogènes, les groupes $-CF_3$, $-C_2F_5$ à $C_{20}$-$F_{41}$, -N(alkyle en $C_1$-$C_{30}$)$_2$, (alkylène en $C_1$-$C_{20}$)-COO-alkyle en $C_1$ à $C_{20}$, hydroxy et hydroxyalkyle, éventuellement en présence d'un ou plusieurs autres composés insaturés, **caractérisé en ce que** l'on polymérise en présence d'un ou plusieurs complexes tungstène-carbyne et/ou molybdène-carbyne, dont le métal central est obligatoirement au stade d'oxydation + VI.

**2.** Procédé selon la revendication 1, **caractérisé en ce que** l'on polymérise en l'absence de solvant.